# EUROPEAN PATENT APPLICATION

(11) **EP 3 827 771 A1**
(43) Date of publication of application: **02.06.2021**
(21) Application number: 18927240.4
(22) Date of filing: 01.08.2018
(51) Int. Cl.: A61B 18/02

(54) **CRYOABLATION DEVICE AND METHOD**

(30) Priority: 23.07.2018 CN 201810812250
(71) Applicant: Piedmont Medsystems (Zhuhai) Co., Ltd., Zhuhai, Guangdong 519031 (CN)
(72) Inventor: XIAO, Jiahua, Zhuhai, Guangdong 519031 (CN); SU, Dongbo, Zhuhai, Guangdong 519031 (CN); DE LA RAMA, Alan, Zhuhai, Guangdong 519031 (CN); HATA, Cary Kunihiko, Zhuhai, Guangdong 519031 (CN)
(74) Representative: Schlich, George
(86) International application number: PCT/CN2018/097905
(87) International publication number: WO 2020/019362

(57) **Abstract**

A cryoablation apparatus comprises a cryoballoon (8), a catheter (7), a storage tank (1), a delivery pipeline (2), a recovery pipeline (3), and a refrigeration assembly. The cryoballoon is provided with a circulating cold carrier medium therein, and the cold carrier medium is a low pressure liquid, a gas or a gas-liquid mixture. A cryoablation method comprises a precooling step, an ablating step, a recovering step, and a rewarming step. The cryoablation apparatus and method provided herein has a low risk factor, is easy to operate and convenient to use, and also has advantages of high utilization efficiency of cold energy and high temperature control accuracy.

## Description

### Technical Field

The present invention relates to the technical field of medical equipments, and particularly relates to a cryoablation apparatus and method.

### Background

Cryoablation is a surgical medical technique to ablate a target tissue by refrigeration, which is usually used in treating diseases such as tumor, atrial fibrillation, etc. Its principle is using a cryogenic device to cause the target tissue to undergo cooling, freezing and rewarming so that irreversible damage or necrosis happens to the cells. A cryoablation apparatus usually includes a cryoablation generator (main body) and a cryoballoon, the cryoablation generator (main body) provides a cold carrier medium to the cryoballoon, and in use, the cryoballoon is mounted on a front end of a catheter which extends into the human body, the cryoablation generator (main body) circulates the cold carrier medium through the catheter into the cryoballoon for cooling the cryoballoon, so as to cryo-ablate a target tissue.

Cryoablation apparatuses in prior art usually adopts a way of refrigeration by high-pressure gas throttling, wherein a high-pressure gas flows through a small hole so as to be adiabatically throttled, as the pressure thereof drops, the temperature thereof would probably change, and thus the adiabatic throttle effect of gas can be utilized to carry out a refrigeration treatment process by cryoablation. However, the way of high-pressure gas throttling has certain defects in actual use: firstly, the high-pressure gas has a high risk factor, and as the cryoballoon at the end of a cryoablation apparatus is usually put inside the human body, once the gas pressure becomes too high, rupture of the cryoballoon may be caused, which greatly threatens the safety of the patient; secondly, the high-pressure gas is consumable and needs to be replenished, which is not convenient for use; moreover, the apparatus has high requirements for an operator, and the operation needs to be performed in the company of a professional technician.

### Summary of the Invention

Thus, a technical problem to be solved by the present invention is how to overcome the defects that the cryoablation apparatuses in prior art has a high risk factor, a fast consumption rate of working medium and inconvenience for use, by providing a cryoablation apparatus that has a low risk factor, a very little consumption rate of working medium and convenience for use.

Another technical problem to be solved by the present invention is how to overcome the defects that the cryoablation methods in prior art has a high risk factor and large operation difficulty, by providing a cryoablation method that has a low risk factor and simplicity for use.

In order to solve the above technical problems, the present application provides the following technical solutions:
A cryoablation apparatus, comprising:
a cryoballoon, provided with a circulating cold carrier medium therein, adapted for contacting a human tissue and performing cryoablation on the human tissue;
a catheter, connected to the cryoballoon, having an inlet end and an outlet end, and adapted for transporting the cold carrier medium into and out of the cryoballoon;
a storage tank, provided with the cold carrier medium stored therein;
a delivery pipeline, having one end communicated with the storage tank and another end communicated with the inlet end of the catheter;
a recovery pipeline, having one end communicated with the storage tank and another end communicated with the outlet end of the catheter; and
a refrigeration assembly, connected in series with the delivery pipeline, and adapted for cooling the cold carrier medium in the delivery pipeline.

As a preferable technical solution, the refrigeration assembly comprises:
a first cold energy exchanger, mounted on the delivery pipeline and used for cooling the cold carrier medium flowing through the first cold energy exchanger by heat exchange;
a cold energy generator, for generating cold energy and providing the cold energy to the first cold energy exchanger.

As a preferable technical solution, the refrigeration assembly further comprises: a second cold energy exchanger, having a hot fluid channel mounted on the delivery pipeline and a cold fluid channel mounted on the recovery pipeline, wherein, cold energy exchange occurs between the cold fluid channel and the hot fluid channel to precool the cold carrier medium flowing through the hot fluid channel, and the hot fluid channel is connected between the storage tank and the first cold energy exchanger.

As a preferable technical solution, the cryoablation apparatus further comprises:
a bypass pipe, communicated with the delivery pipeline and the recovery pipeline to allow the delivery pipeline and the recovery pipeline to form a precooling looping path connecting the storage tank and the first cold energy exchanger in series;
wherein, the bypass pipe is communicated with the delivery pipeline via a first three-way valve.

As a preferable technical solution, the refrigeration assembly further comprises:
a cold storage device, mounted on the recovery pipeline, communicated with the first cold energy exchanger via the bypass pipe, and adapted for storing cold energy coming from the first cold energy exchanger.

As a preferable technical solution, the refrigeration assembly further comprises:
a heat-insulation device, having a heat-insulation chamber adapted for reducing or eliminating heat conduction to the exterior thereof, with the first cold energy exchanger, the second cold energy exchanger, the cold storage device and a cold energy output end of the cold energy generator located inside the heat-insulation chamber.

As a preferable technical solution, the heat-insulation device is a box mounted with a vacuumizing device communicated with the heat-insulation chamber.

As a preferable technical solution, the heat-insulation device is a box with the heat-insulation chamber thereof filled with heat insulation substance.

As a preferable technical solution, the cryoablation apparatus further comprises a rewarming looping path for transporting the cold carrier medium in the storage tank to the inlet end of the catheter of the cryoablation apparatus.

As a preferable technical solution, the rewarming looping path comprises:
a rewarming pipe;
wherein, an inlet end of the rewarming pipe is connected by a second three-way valve to a side of the delivery pipeline that is located upstream of the first cold energy exchanger.

As a preferable technical solution, the cryoablation apparatus further comprises a rewarming looping path for transporting the cold carrier medium in the storage tank to the inlet end of the catheter of the cryoablation apparatus after heating the cold carrier medium.

As a preferable technical solution, the rewarming looping path comprises:
a rewarming pipe with a heating device connected in series;
wherein, an inlet end of the rewarming pipe is connected by a second three-way valve to an upstream side of an inlet to the hot fluid channel.

As a preferable technical solution, the rewarming looping path further comprises:
a rewarming backflow pipeline for connecting the outlet end of the catheter of the cryoablation apparatus to the storage tank.

As a preferable technical solution, the rewarming backflow pipeline comprises:
a rewarming backflow pipe having both ends thereof communicated with the recovery pipeline and being connected in parallel to the second cold energy exchanger;
wherein, an inlet of the rewarming backflow pipe is connected by a third three-way valve to the delivery pipeline.

As a preferable technical solution, the cryoablation apparatus further comprises:
a heat-insulation device, having a heat-insulation chamber adapted for reducing or eliminating heat conduction to the exterior thereof, with the first cold energy exchanger, the second cold energy exchanger, a cold storage device and a cold energy output end of the cold energy generator located inside the heat-insulation chamber;
wherein, the rewarming backflow pipe is located outside the heat-insulation device.

As a preferable technical solution, a pumping device is connected in series on the delivery pipeline or the recovery pipeline, the pumping device is adapted for providing motive power for the flowing of the cold carrier medium.

A cryoablation method, comprising:
a precooling step, in which a cold carrier medium is circulated through a cold energy generator for cooling;
an ablating step, in which the precooled cold carrier medium is re-circulated through the cold energy generator for re-cooling and then is transported to a target tissue of a human body, so that cold energy exchange occurs between the cold carrier medium and the target tissue to cool the target tissue for cryoablation of the target tissue;
a recovering step, in which the cold carrier medium after cold energy exchange with the target tissue is transported out of the human body and into a storage tank; and
a rewarming step, in which the transportation of the cooled cold carrier medium into the human body is stopped, and the target tissue is rewarmed.

As a preferable technical solution, in the recovering step, the cold carrier medium after cold energy exchange with the target tissue is transported out of the human body, and residual cold energy in the cold carrier medium transported out of the human body is utilized to carry out cold energy exchange with the cold carrier medium flowing out of the storage tank at a second cold energy exchanger, so as to cause the cold carrier medium before entry into the first cold energy exchanger to be cooled and then transported into the first cold energy exchanger.

As a preferable technical solution, a part of cold energy is stored in the precooling stage, and then transferred to the cold carrier medium recovered from a catheter, so as to be exchanged to the cold carrier medium flowing out of the storage tank at a second cold energy exchanger.

As a preferable technical solution, in the rewarming step, the cold carrier medium is circulated through a heating device for being warmed, and then the cold carrier medium after being warmed is transported to the target tissue of the human body, so as to warm the cooled target tissue by heat exchange between the cold carrier medium and the target tissue.

As a preferable technical solution, in the rewarming step, uncooled cold carrier medium is transported to the human body, so as to warm the target tissue by heat exchange between the uncooled cold carrier medium and the target tissue.

The technical solutions of the present invention have the following advantages:
1. The cryoablation apparatus provided by the present invention comprises a cryoballoon, a catheter, a storage tank, a delivery pipeline, a recovery pipeline, and a refrigeration assembly; the cryoballoon is provided with a circulating cold carrier medium therein and adapted for contacting a human tissue and performing cryoablation on the human tissue; the catheter is connected to the cryoballoon, has an inlet end and an outlet end, and is adapted for transporting the cold carrier medium into and out of the cryoballoon; the storage tank has the cold carrier medium stored therein, and the cold carrier medium is a low pressure liquid, a gas or a gas-liquid mixture; the delivery pipeline has one end communicated with the storage tank and another end communicated with the inlet end of the catheter; the recovery pipeline has one end communicated with the storage tank and another end communicated with the outlet end of the catheter; and the refrigeration assembly is connected in series with the delivery pipeline, and is adapted for providing cold energy to the cold carrier medium in the delivery pipeline. In a process of cryoablation operation, the cold carrier medium flows in accordance with the following path: flowing out from the storage tank along the delivery pipeline, passing through the refrigeration assembly where the temperature of the cold carrier medium is lowered, then entering the inlet end of the catheter, flowing into the cryoballoon so as to contact the target tissue via the cryoballoon, then flowing out from the outlet end of the catheter and into the recovery pipeline, and finally returning to the storage tank to complete a circulation cycle. In the above process, because the previous high-pressure gas is replaced with the cold carrier medium which is directly cooled for refrigeration, the low temperature requirement for cryoablation can be met; the cold carrier medium, as compared to high-pressure gas, is less likely to cause explosion, so that the risk factor during use of the cryoablation apparatus is effectively reduced; meanwhile, as the cold carrier medium can be recycled for reuse, there is no need for replenishment in a process of use, so that its convenience for use is improved. Also, the low-pressure cold carrier medium has a simple way of cooling, only relying on the refrigeration assembly, therefore, its cooling process is easier to control than the way of cooling by relying on high-pressure gas throttling, hence, its operation difficulty level is effectively lowered, and the entire operation process can be performed by a surgeon without the company of a professional technician.
2. In the cryoablation apparatus provided by the present invention, the refrigeration assembly further comprises a second cold energy exchanger having a hot fluid channel mounted on the delivery pipeline and a cold fluid channel mounted on the recovery pipeline, wherein, cold energy exchange occurs between the cold fluid channel and the hot fluid channel to precool the cold carrier medium flowing through the hot fluid channel, and the hot fluid channel is connected between the storage tank and the first cold energy exchanger. When carrying out cryoablation, the delivery pipeline, the recovery pipeline together with the catheter and the cryoballoon of the cryoablation apparatus are used to circulate the cold carrier medium to flow, and during the flow process, the cold energy generated at the cold energy generator is transferred to the delivery pipeline through the first cold energy exchanger, and then transferred into the human body through the catheter for cryo-ablating a target tissue, and after cold energy exchange with the target tissue, the cold carrier medium subsequently flows back to the recovery pipeline through the catheter, and at this time, the cold carrier medium still has a residual part of cold energy, then, as the cold carrier medium flows through the recovery pipeline, because of the existence of the second cold energy exchanger, the residual part of cold energy in the cold carrier medium in the recovery pipeline is transferred to the cold carrier medium in the delivery pipeline via the second cold energy exchanger, thereby causing the temperature of the cold carrier medium in the delivery pipeline to drop beforehand. In the above process, because, on the delivery pipeline, the second cold energy exchanger is on an upstream side relative to the first cold energy exchanger where the cold carrier medium in the delivery pipeline has a temperature higher than that of the cold carrier medium in the recovery pipeline, it can therefore be ensured that cold energy is transferred from the recovery pipeline to the delivery pipeline. By the above process, the residual cold energy of the cold carrier medium in the recovery pipeline can precool the cold carrier medium to reduce the initial temperature of the cold carrier medium entering the first cold energy exchanger, therefore, under the condition of the same cold energy exchange amount, such precooled cold carrier medium can reach a lower temperature, so that through the cryoablation by directly refrigerating a low-pressure medium, the temperature required for cryoablation is more likely to be reached, meanwhile, the utilization efficiency of cold energy is improved.
3. The cryoablation apparatus provided by the present invention further comprises a bypass pipe, communicated with the delivery pipeline and the recovery pipeline to allow the delivery pipeline and the recovery pipeline to form a precooling looping path connecting the storage tank and the first cold energy exchanger in series; wherein, the bypass pipe is communicated with the delivery pipeline via a first three-way valve. By using the bypass pipe, the cold carrier medium can be precooled before being circulated into the human body for carrying out cryoablation, wherein, in a precooling step, the cold carrier medium comes out from the storage tank, sequentially flows through the delivery pipeline, the first cold energy exchanger, the bypass pipe and the recovery pipeline, and finally goes back into the storage tank. The precooled cold carrier medium has a reduced temperature to provide a lower initial temperature for entering the cryo-ablating step, and thus the low temperature required for cryoablation can be more easily reached after re-cooling by the first cold energy exchanger. Therefore, this measure can further increase the likelihood for the cryoablation apparatus to make the cold carrier medium reach the temperature required for cryoablation.
4. The cryoablation apparatus provided by the present invention further comprises a cold storage device mounted on the recovery pipeline, communicated with the first cold energy exchanger via the bypass pipe, and adapted for storing cold energy coming from the first cold energy exchanger. In the precooling step, the cold storage device can store a part of cold energy brought by the cold carrier medium, and after the cryo-ablating step has started, this stored part of cold energy can precool the cold carrier medium flowing out of the human body, so as to increase the temperature difference between the cold fluid channel and the hot fluid channel at the second cold energy exchanger, increase the cold energy exchange rate at the second cold energy exchanger, and thus further reduce the temperature of the cold carrier medium in the delivery pipeline, and this precooled cold carrier medium can reach a lower temperature after final cooling by the first cold energy exchanger. Therefore, this measure can further ensure that the cryoablation apparatus can reach the low temperature required for cryoablation, and can further improve the efficiency of utilization of cold energy and reduce waste of cold energy.
5. The cryoablation apparatus provided by the present invention further comprises a heat-insulation device which has a heat-insulation chamber adapted for reducing or eliminating heat conduction to the exterior thereof, with the first cold energy exchanger, the second cold energy exchanger, the cold storage device and a cold energy output end of the cold energy generator located inside the heat-insulation chamber. By using the heat-insulation device, loss of cold energy can be prevented during the cold energy exchanging process, meanwhile, the heat preservation effect of the cold storage device is better, as loss of cold energy from the cold storage device is prevented during the cold energy storing process.
6. In the cryoablation apparatus provided by the present invention, the heat-insulation device is a box mounted with a vacuumizing device communicated with the heat-insulation chamber. The heat-insulation chamber in a near-vacuum state can further reduce the loss rate of cold energy, and thus help the cryoablation apparatus further improve the efficiency of utilization of cold energy.
7. The cryoablation apparatus provided by the present invention further comprises a rewarming looping path for transporting the cold carrier medium in the storage tank to the inlet end of the catheter of the cryoablation apparatus after heating the cold carrier medium. In a cryoablation operation, the refrigerated target tissue needs to be rewarmed, and an ideal rewarming process can improve the operative effect of the cryoablation operation and reduce the probability of occurrence of any postoperative complication. The rewarming looping path provided in the present invention can heat the cold carrier medium and transport the cold carrier medium to the target tissue through the catheter, such a separately arranged rewarming looping path not only can meet the demands of rewarming for the cryoablation operation, but also is very beneficial for accurately controlling the temperature, progress and duration of the rewarming, thereby increasing surgical cure rate and reducing postoperative complications.
8. In the cryoablation apparatus provided by the present invention, the rewarming looping path comprises a rewarming pipe with a heating device connected in series; wherein, an inlet end of the rewarming pipe is connected by a second three-way valve to an upstream side of an inlet to the hot fluid channel. By connecting the rewarming pipe to the upstream side of the second cold energy exchanger on the delivery pipeline, the rewarming pipe is connected in parallel to the first cold energy exchanger and the second cold energy exchanger, so that the pipeline for heating the cold carrier medium is independent from the pipeline for cooling the cold carrier medium. Therefore, interference to the heating process of the cold carrier medium caused by residual cold energy of the first cold energy exchanger and the second cold energy exchanger can be prevented in the rewarming step, thereby reducing interference factors in the rewarming process, and making it easier to control the rewarming process.
9. In the cryoablation apparatus provided by the present invention, the rewarming looping path further comprises a rewarming backflow pipeline for connecting the outlet end of the catheter of the cryoablation apparatus to the storage tank. The separately arranged rewarming backflow pipeline can make the rewarming process form an independent rewarming loop composed of the storage tank, the rewarming pipe, the cryoablation apparatus, and the rewarming backflow pipeline, thereby further reducing interference factors in the rewarming process, and making the control of the rewarming process more accurate.
10. The cryoablation apparatus provided by the present invention further comprises a heat-insulation device which has a heat-insulation chamber adapted for reducing or eliminating heat conduction to the exterior thereof, with the first cold energy exchanger, the second cold energy exchanger, a cold storage device and a cold energy output end of the cold energy generator located inside the heat-insulation chamber, wherein the rewarming backflow pipe is located outside the heat-insulation device. As the rewarming backflow pipe is arranged outside the heat-insulation device, it can be prevented that the cold energy in the cold storage device or the second cold energy exchanger is brought away by the rewarming backflow pipe when transporting the cold carrier medium after rewarming, so as to improve the utilization rate of cold energy.
11. The technical solutions provided by the present invention also include a cryoablation method comprising: a precooling step, in which a low-pressure cold carrier medium is circulated through a cold energy generator for cooling; an ablating step, in which the precooled cold carrier medium is re-circulated through the cold energy generator and then is transported to a target tissue of the human body, so that cold energy exchange occurs between the cold carrier medium and the target tissue to cool the target tissue for cryoablation of the target tissue; a recovering step, in which the cold carrier medium after cold energy exchange with the target tissue is transported out of the human body and into a storage tank; and a rewarming step, in which the transportation of the cooled cold carrier medium into the human body is stopped, and the target tissue is rewarmed. In the above steps, because there is the precooling step, the cold carrier medium after being precooled has a lower temperature, so as to provide a lower initial temperature for the refrigerating process of the ablating step, and therefore a lower final temperature can be reached. As compared to the conventional way of directly refrigerating a cold carrier medium and then passing the cold carrier medium into a human body, in this method, it is easier for the cold carrier medium to reach the low temperature required for cryoablation.
12. In the cryoablation method provided by the present invention, in the recovering step, the cold carrier medium after cold energy exchange with the target tissue is transported out of the human body, and residual cold energy in the cold carrier medium transported out of the human body is utilized to carry out cold energy exchange with the cold carrier medium before entry into the cold energy generator, so as to cause the cold carrier medium before entry into the cold energy generator to be cooled and then transported to the cold energy generator. In the above step, the residual cold energy of the cold carrier medium in the recovery pipeline can be reused, thereby improving the efficiency of utilization of cold energy and reducing the power burden of the cold energy generator.
13. In the cryoablation method provided by the present invention, a part of cold energy is stored in the precooling stage, and then transferred to the cold carrier medium transported out of the human body, so as to cool the cold carrier medium transported out of the human body. By the above step, the pre-stored cold energy after being transferred to the recovery pipeline can reduce the temperature of the recovered cold carrier medium, so as to increase the temperature difference in the process of cooling the cold carrier medium to be inputted by utilizing the residual cold energy in the cold carrier medium outputted from the human body, increase the cold energy exchange rate between the two, and thus further reduce the temperature of the cold carrier medium to be inputted into the human body, so that it is easier to reach the temperature required for cryoablation with the refrigerating of the cold energy generator.

In conclusion, the cryoablation apparatus and method provided by the present invention has a low risk factor, is easy to operate and convenient to use, and also has advantages of high utilization efficiency of cold energy and temperature control accuracy.

### Brief Description of the Drawings

In order to describe the technical solutions more clearly in the specific embodiments of the present application or in the prior art, hereinafter, the appended drawings used for describing the specific embodiments or the prior art will be briefly introduced. Apparently, the drawings described below show only some embodiments of the present application, and for a person skilled in the art, without expenditure of creative labor, other drawings can be derived on the basis of these appended drawings.
FIG. 1 is a structural schematic diagram of a cryoablation apparatus provided in Embodiment 1 of the present invention;
FIG. 2 is a schematic diagram of the flow direction of the cold carrier medium in the precooling step for the cryoablation apparatus of FIG. 1;
FIG. 3 is a schematic diagram of the flow direction of the cold carrier medium in the cryo-ablating step for the cryoablation apparatus of FIG. 1;
FIG. 4 is a schematic diagram of the flow direction of the cold carrier medium in the rewarming step for the cryoablation apparatus of FIG. 1;
FIG. 5 is a flow chart of a cryoablation method provided in Embodiment 2 of the present invention.

### Reference signs:

1-storage tank, 2-delivery pipeline, 3-recovery pipeline, 4-cold energy generator, 5-first cold energy exchanger, 6-second cold energy exchanger, 7-catheter, 8-cryoballoon, 9-bypass pipe, 10-first three-way valve, 11-cold storage device, 12-heat-insulation device, 13-heat-insulation chamber, 14-vacuumizing device, 15-rewarming pipe, 16-heating device, 17-second three-way valve, 18-rewarming backflow pipe, 19-third three-way valve, 20-pumping device, 21-flowmeter, 22-thermometer, 23-one-way valve, 24-heat radiator.

### Detailed Description of Embodiments

A clear and complete description of the technical solutions of the present invention is given below, in conjunction with the appended drawings. Apparently, the described embodiments are a part, but not all, of the embodiments of the present invention. All the other embodiments, derived by a person skilled in the art on the basis of the embodiments described in the present invention without expenditure of creative labor, are included in the protection scope of the present invention.

In the description of the present invention, it needs to be noted that, terms such as "center", "above", "below", "left", "right", "vertical", "horizontal", "inside", "outside" refer to the orientation or positional relation based on the illustration of the drawings, which is merely for facilitating and simplifying the description of the present application, not for indicating or implying that the referred apparatus or component must have a particular orientation or must be configured or operated in a particular orientation, therefore is not to be construed as a limitation towards the present invention. In addition, terms such as "first", "second", "third" are merely for the purpose of description, and are not to be construed as an indication or implication of relative importance thereof.

In the description of the present invention, it needs to be noted that, unless specifically defined or restricted otherwise, terms such as "mount", "interconnect", "connect" should be broadly construed, for example, they may be fixed connection or detachable connection or integral connection; they may be mechanical connection or electrical connection; they may be direct connection, or indirect connection via an intermediate medium, or internal communication between two units. For a person skilled in the art, the specific meaning of the afore-mentioned terms in the present invention can be understood according to specific situations thereof.

Furthermore, the technical features involved in different embodiments of the present invention described below can be combined with one another as long as they do not conflict with one another.

### Embodiment 1

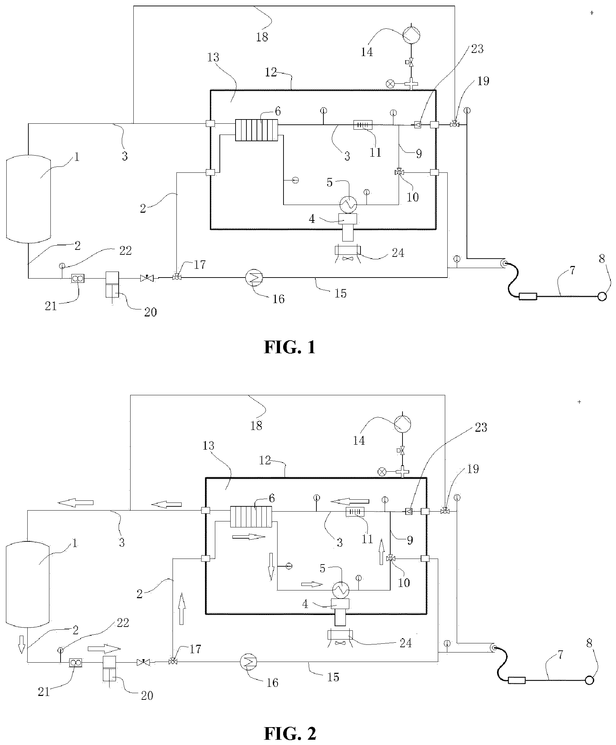

FIG. 1 to FIG. 4 show Embodiment 1 of the present invention, the present embodiment provides a cryoablation apparatus which comprises a cryoballoon 8, a catheter 7, a storage tank 1, a delivery pipeline 2, a recovery pipeline 3, and a refrigeration assembly; the cryoballoon 8 is provided with a circulating cold carrier medium therein and adapted for contacting a human tissue and performing cryoablation on the human tissue; the catheter 7 is connected to the cryoballoon 8, has an inlet end and an outlet end, and is adapted for transporting the cold carrier medium into and out of the cryoballoon 8; the storage tank 1 has the cold carrier medium stored therein, and the cold carrier medium is a low-pressure medium; the delivery pipeline 2 has one end communicated with the storage tank 1 and another end communicated with the inlet end of the catheter 7; the recovery pipeline 3 has one end communicated with the storage tank 1 and another end communicated with the outlet end of the catheter 7; and the refrigeration assembly is connected in series with the delivery pipeline 2, and is adapted for cooling the cold carrier medium in the delivery pipeline 2.

In a process of cryoablation operation, the cold carrier medium flows in accordance with the following path: flowing out from the storage tank 1 along the delivery pipeline 2, passing through the refrigeration assembly where the temperature of the cold carrier medium is lowered, then entering the inlet end of the catheter 7, flowing into the cryoballoon 8 so as to contact the target tissue via the cryoballoon, then flowing out from the outlet end of the catheter 7 and into the recovery pipeline 3, and finally returning to the storage tank 1 to complete a circulation cycle. In the above process, because the previous high-pressure gas is replaced with the cold carrier medium which is directly cooled for refrigeration and is less likely to cause explosion as compared to high-pressure gas, so that the risk factor during use of the cryoablation apparatus is effectively reduced, meanwhile, as the low-pressure cold carrier medium can be recycled for reuse, there is no need for replenishment in a process of use, so that its convenience for use is improved. Also, the low-pressure cold carrier medium has a simple way of cooling, only relying on the refrigeration assembly, therefore, its cooling process is easier to control than the way of cooling by relying on high-pressure gas throttling, hence, its operation difficulty level is effectively lowered, and the entire operation process can be performed by a surgeon without the company of a professional technician.

As a specific embodiment of the refrigeration assembly, the refrigeration assembly comprises a cold energy generator 4, a first cold energy exchanger 5, and a second cold energy exchanger 6. The first cold energy exchanger 5 is mounted on the delivery pipeline 2 and used for cooling the cold carrier medium flowing through the first cold energy exchanger 5 by heat exchange; the cold energy generator 4 provides cold energy to the first cold energy exchanger 5; the second cold energy exchanger 6 has a hot fluid channel mounted on the delivery pipeline 2 and a cold fluid channel mounted on the recovery pipeline 3, wherein, cold energy exchange occurs between the cold fluid channel and the hot fluid channel to precool the cold carrier medium flowing through the hot fluid channel, and the hot fluid channel is connected between the storage tank 1 and the first cold energy exchanger 5.

When the above refrigeration assembly participates in cryoablation, the delivery pipeline 2, the recovery pipeline 3 together with the catheter 7 and the cryoballoon 8 of the cryoablation apparatus are used to circulate the cold carrier medium to flow, and during the flow process, the cold energy generated at the cold energy generator 4 is transferred to the delivery pipeline 2 through the first cold energy exchanger 5, and then transferred into a human body through the catheter 7 for cryo-ablating a target tissue, and after cold energy exchange with the target tissue, the cold carrier medium subsequently flows back to the recovery pipeline 3 through the catheter 7, and at this time, the cold carrier medium still has a residual part of cold energy, then, as the cold carrier medium flows through the recovery pipeline 3, because of the existence of the second cold energy exchanger 6, the residual part of cold energy in the cold carrier medium in the recovery pipeline 3 is transferred to the cold carrier medium in the delivery pipeline 2 via the second cold energy exchanger 6, thereby causing the temperature of the cold carrier medium in the delivery pipeline 2 to drop beforehand.

In the above process, because, on the delivery pipeline 2, the second cold energy exchanger 6 is on an upstream side relative to the first cold energy exchanger 5 where the cold carrier medium in the delivery pipeline 2 has a temperature higher than that of the cold carrier medium in the recovery pipeline 3, it can therefore be ensured that cold energy is transferred from the recovery pipeline 3 to the delivery pipeline 2. By the above process, the residual cold energy of the cold carrier medium in the recovery pipeline 3 can precool the cold carrier medium to reduce the initial temperature of the cold carrier medium entering the first cold energy exchanger 5, therefore, under the condition of the same cold energy exchange amount, such precooled cold carrier medium can reach a lower temperature, so that through the cryoablation by directly refrigerating a low-pressure medium, the temperature required for cryoablation is more likely to be reached, meanwhile, the utilization efficiency of cold energy is improved.

Specifically, the cold energy generator 4 in the present embodiment is particularly a miniature ultralow-temperature refrigerator that can provide a cold source below -120°C, in the form of a pulse tube, a Stirling engine, a hybrid throttling device, a thermoacoustic device, etc., and there may be one device or multiple devices, and when multiple devices work in combination, they may be combined by being connected in series or in parallel. The cold carrier medium in the present embodiment is a liquid having a low solidification point, for example, absolute ethyl alcohol.

In order to further reduce the lowest temperature that can be reached by the cold carrier medium, a bypass pipe 9 is further comprised, the bypass pipe 9 is communicated with the delivery pipeline 2 and the recovery pipeline 3 to allow the delivery pipeline 2 and the recovery pipeline 3 to form a precooling looping path connecting the storage tank 1 and the first cold energy exchanger 5 in series; and the bypass pipe 9 is communicated with the delivery pipeline 2 via a first three-way valve 10.

By using the bypass pipe 9, the cold carrier medium can be precooled before being circulated into the human body for carrying out cryoablation, wherein, in a precooling step, the cold carrier medium comes out from the storage tank 1, sequentially flows through the delivery pipeline 2, the first cold energy exchanger 5, the bypass pipe 9 and the recovery pipeline 3, and finally goes back into the storage tank 1. The precooled cold carrier medium has a reduced temperature to provide a lower initial temperature for entering the cryo-ablating step, and thus the low temperature required for cryoablation can be more easily reached after re-cooling by the first cold energy exchanger 5. Therefore, this measure can further increase the likelihood for the refrigeration equipment to make the cold carrier medium reach the temperature required for cryoablation.

As an improved embodiment of the cryoablation apparatus, a cold storage device 11 is further comprised, the cold storage device 11 is mounted on the recovery pipeline 3, communicated with the first cold energy exchanger 5 via the bypass pipe 9, and adapted for storing cold energy coming from the first cold energy exchanger 5. In this embodiment, the cold storage device 11 is specifically a box filled with a cold storage medium having a high specific heat capacity, the recovery pipeline 3 passes through the cold storage device 11 and utilizes a side wall of the pipeline to carry out cold energy exchange with the cold storage medium in the cold storage device 11.

In the precooling step, the cold storage device 11 can store a part of cold energy brought by the cold carrier medium, and after the cryo-ablating step has started, this stored part of cold energy can precool the cold carrier medium flowing out of the human body, so as to increase the temperature difference between the cold fluid channel and the hot fluid channel at the second cold energy exchanger 6, increase the cold energy exchange rate at the second cold energy exchanger 6, and thus further reduce the temperature of the cold carrier medium in the delivery pipeline 2, and this precooled cold carrier medium can reach a lower temperature after final cooling by the first cold energy exchanger 5. Therefore, this measure can further ensure that the cryoablation apparatus can reach the low temperature required for cryoablation, and can further improve the efficiency of utilization of cold energy and reduce waste of cold energy.

In order reduce loss of cold energy, a heat-insulation device 12 is further comprised, the heat-insulation device 12 has a heat-insulation chamber 13 adapted for reducing or eliminating heat conduction to the exterior thereof, with the first cold energy exchanger 5, the second cold energy exchanger 6, the cold storage device 11 and a cold energy output end of the cold energy generator 4 located inside the heat-insulation chamber 13. By using the heat-insulation device 12, loss of cold energy can be prevented during the cold energy exchanging process, meanwhile, the heat preservation effect of the cold storage device 11 is better, as loss of cold energy from the cold storage device 11 is prevented during the cold energy storing process.

Specifically, the heat-insulation device 12 is a box mounted with a vacuumizing device 14 communicated with the heat-insulation chamber 13. The heat-insulation chamber 13 in a near-vacuum state can further reduce the loss rate of cold energy, and thus help the refrigeration equipment further improve the efficiency of utilization of cold energy. The vacuumizing device 14 is a miniature vacuum pump.

As an alternative of the heat-insulation device, the heat-insulation device 12 is a box with the heat-insulation chamber 13 thereof filled with heat insulation substance. Herein, the heat insulation substance may use a heat insulation material such as a polyurethane foamed material or an aerogel.

In order to meet the demands for rewarming the target tissue after refrigerating, the present embodiment also comprises a rewarming looping path for transporting the cold carrier medium in the storage tank to the liquid inlet of the catheter 7 of the cryoablation apparatus after heating the cold carrier medium. In a cryoablation operation, the refrigerated target tissue needs to be rewarmed, and an ideal rewarming process can improve the operative effect of the cryoablation operation and reduce the probability of occurrence of any postoperative complication. The rewarming looping path provided in the present application can heat the cold carrier medium and transport the cold carrier medium to the target tissue through the catheter 7, such a separately arranged rewarming looping path not only can meet the demands of rewarming for the cryoablation operation, but also is very beneficial for accurately controlling the temperature, progress and duration of the rewarming, thereby increasing surgical cure rate and reducing postoperative complications.

Specifically, the rewarming looping path comprises a rewarming pipe 15 with a heating device 16 connected in series, wherein an inlet end of the rewarming pipe 15 is connected by a second three-way valve 17 to an upstream side of an inlet to the hot fluid channel. By connecting the rewarming pipe 15 to the upstream side of the second cold energy exchanger 6 on the delivery pipeline 2, the rewarming pipe 15 is connected in parallel to the first cold energy exchanger 5 and the second cold energy exchanger 6, so that the pipeline for heating the cold carrier medium is independent from the pipeline for cooling the cold carrier medium. Therefore, interference to the heating process of the cold carrier medium caused by residual cold energy of the first cold energy exchanger 5 and the second cold energy exchanger 6 can be prevented in the rewarming step, thereby reducing interference factors in the rewarming process, and making it easier to control the rewarming process.

As an alternative to the above embodiment of rewarming looping path, the rewarming looping path comprises a rewarming pipe 15, wherein an inlet end of the rewarming pipe 15 is connected by a second three-way valve 17 to a side of the delivery pipeline 2 that is located upstream of the first cold energy exchanger 5. In this alternative embodiment, there is no heating device connected in series on the rewarming looping path, and instead, the uncooled cold carrier medium is passed into the catheter for participating in the rewarming process, wherein native heat energy of the human body is utilized to perform rewarming. This measure makes the warming of the target tissue more moderate, thereby reducing any damage caused by the cryoablation operation to healthy tissues.

As a further improvement of the rewarming looping path, the rewarming looping path comprises a rewarming backflow pipeline for connecting the liquid outlet of the catheter 7 of the cryoablation apparatus to a return port of the storage tank 1. The separately arranged rewarming backflow pipeline can make the rewarming process form an independent rewarming loop composed of the storage tank 1, the rewarming pipe 15, the cryoablation apparatus, and the rewarming backflow pipeline, thereby further reducing interference factors in the rewarming process, and making the control of the rewarming process more accurate.

Specifically, the rewarming backflow pipeline comprises a rewarming backflow pipe 18 having both ends thereof communicated with the recovery pipeline 3 and being connected in parallel to the second cold energy exchanger 6, wherein an inlet of the rewarming backflow pipe 18 is connected by a third three-way valve 19 to the delivery pipeline 2. Furthermore, the rewarming backflow pipe 18 is located outside the heat-insulation device 12. As the rewarming backflow pipe 18 is arranged outside the heat-insulation device 12, it can be prevented that the cold energy in the cold storage device 11 or the second cold energy exchanger 6 is brought away by the rewarming backflow pipe 18 when transporting the cold carrier medium after rewarming, so as to improve the utilization rate of cold energy.

In order to ensure smooth circulation of the cold carrier medium, a pumping device 20 is connected in series on the delivery pipeline 2 or the recovery pipeline 3, the pumping device 20 is adapted for providing motive power for the flowing of the cold carrier medium.

The cryoablation apparatus provided in the present embodiment also comprises an operating device. The operating device comprises a handle and an actuator, for manipulating the catheter 7 to reach the target tissue to be ablated. When performing a cryoablation operation, the catheter 7 is a pipeline for transporting the low-temperature cold carrier medium, and is made of a material having certain tenacity, a low thermal conductivity and physiological compatibility, with multiple channels provided therein, including a cold carrier medium input channel, a cold carrier medium output channel, a functional channel and isolation chambers. The cold carrier medium input channel and output channel are disposed on two lateral sides, so as for heat insulation between the input fluid and the output fluid, and for preventing heat short-circuit. The functional channel is in the center of the catheter 7, for wiring of functional components such as sensors and a guidewire. The isolation chambers are disposed on both ends of the cold carrier medium channels, for further reducing heat exchange between the input fluid and the output fluid. A heat insulation material is wrapped on an exterior of the catheter 7, for reducing heat conduction between the cold carrier medium in the catheter 7 and external human tissues, so that, in on aspect, heat leakage of the cold carrier medium is reduced, and in another aspect, the outer wall temperature of the catheter 7 is prevented from becoming so low that tissue freezing is caused. The cryoballoon 8 is used to cryo-ablate the target tissue and internally has an inlet, connected to the input channel of the catheter 7, and an outlet, connected to the output channel of the catheter 7. After the balloon gets in contact with the tissue, the cold carrier medium exchanges heat with the tissue via the balloon wall.

### Embodiment 2

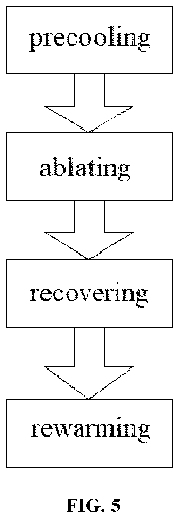

FIG. 5 show Embodiment 2 of the present invention, the present embodiment provides a cryoablation method, which utilizes a low-pressure cold carrier medium and, after refrigerating, directly transports the cold carrier medium into a human body to cryo-ablate a target tissue of the human body. The method specifically comprises the following steps:
a precooling step, in which a cold carrier medium is circulated through a cold energy generator 4 for cooling;
an ablating step, in which the precooled cold carrier medium is re-circulated through the cold energy generator 4 and then is transported to a target tissue of a human body, so that cold energy exchange occurs between the cold carrier medium and the target tissue to cool the target tissue for cryoablation of the target tissue;
a recovering step, in which the cold carrier medium after cold energy exchange with the target tissue is transported out of the human body and into a storage tank; and
a rewarming step, in which the transportation of the cooled cold carrier medium into the human body is stopped, and the target tissue is rewarmed.

In the above steps, because there is the precooling step, the cold carrier medium after being precooled has a lower temperature, so as to provide a lower initial temperature for the refrigerating process of the ablating step, and therefore a lower final temperature can be reached. As compared to the conventional way of directly refrigerating a cold carrier medium and then passing the cold carrier medium into the human body, in this method, it is easier for the cold carrier medium to reach the low temperature required for cryoablation.

In the recovering step, the cold carrier medium after cold energy exchange with the target tissue is transported out of the human body, and residual cold energy in the cold carrier medium transported out of the human body is utilized to carry out cold energy exchange with the cold carrier medium before entry into the cold energy generator 4, so as to cause the cold carrier medium before entry into the cold energy generator 4 to be cooled and then transported to the cold energy generator 4. In the recovering step, the residual cold energy of the cold carrier medium in the recovery pipeline 3 can be reused, thereby improving the efficiency of utilization of cold energy and reducing the power burden of the cold energy generator 4.

A part of cold energy is stored in the precooling stage, and then transferred to the cold carrier medium transported out of the human body, so as to cool the cold carrier medium transported out of the human body. By the above step, the pre-stored cold energy after being transferred to the recovery pipeline 3 can reduce the temperature of the recovered cold carrier medium, so as to increase the temperature difference in the process of cooling the cold carrier medium to be inputted by utilizing the residual cold energy in the cold carrier medium outputted from the human body, increase the cold energy exchange rate between the two, and thus further reduce the temperature of the cold carrier medium to be inputted into the human body, so that it is easier to reach the temperature required for cryoablation with the refrigerating of the cold energy generator 4.

As a preferable embodiment of the rewarming step, in the rewarming step, the cold carrier medium is circulated through a heating device 16 for being warmed, and then the cold carrier medium after being warmed is transported to the target tissue of the human body, so as to warm the cooled target tissue by heat exchange between the cold carrier medium and the target tissue.

As a preferable embodiment of the rewarming step, in the rewarming step, uncooled cold carrier medium is transported to the human body, so as to warm the target tissue by heat exchange between the uncooled cold carrier medium and the target tissue.

In combination with the cryoablation apparatus described in Embodiment 1, the cryoablation method in the present embodiment has the following specific procedure:
In the precooling step, as shown in FIG. 2, the cold energy generator 4 and the pumping device 20 is turned on, and the three three-way valves are adjusted to make the cold carrier medium flow in accordance with the following path: the cold carrier medium at ambient temperature flows out from the storage tank 1, through a flowmeter, the pumping device 20, a flow rate regulation valve and then into the heat-insulation device 12, and after precooling by heat exchange with backflowing cold carrier medium at the second cold energy exchanger 6, the cold carrier medium enters the first cold energy exchanger 5 for cooling, then flows through the bypass pipe 9 and into the cold storage device 11 where a part of cold energy is stored into the cold storage device 11, then backflows to the second cold energy exchanger 6 to precool the cold carrier medium flowing out from the storage tank 1, then flows out of the heat-insulation device 12, and finally returns to the storage tank 1.

After performing the precooling cycle for about 20 minutes, the cold carrier medium drops to minus 80-100°C.

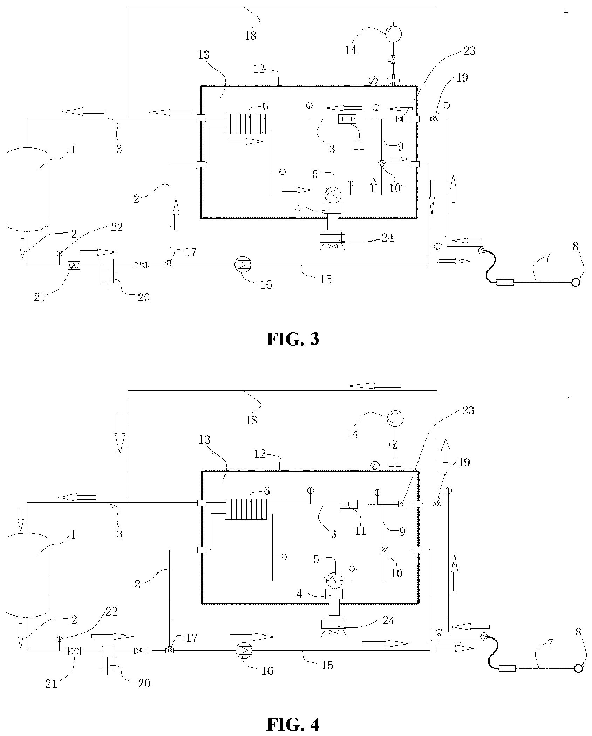

In the ablating step, as shown in FIG. 3, after the precooling is completed, the three three-way valves are adjusted to make the cold carrier medium flow in accordance with the following path: flowing through the flowmeter, the pumping device 20, the flow rate regulation valve and then into the heat-insulation device 12, and after heat exchange with backflowing cold carrier medium at the second cold energy exchanger 6, entering the first cold energy exchanger 5 for cooling, then flowing out of the heat-insulation device 12 and into the catheter 7, then entering the cryoballoon 8 for ablation, then flowing out of the catheter 7 and back into the heat-insulation device 12, flowing through the cold storage device 11 where the temperature of the cold carrier medium is higher than the temperature of the heat storage medium inside the cold storage device 11 so that the cold carrier medium release heat to the heat storage medium and is cooled, then entering the second cold energy exchanger 6 to exchange cold energy with the cold carrier medium flowing out from the storage tank 1, then flowing out of the heat-insulation device 12 and returning to the storage tank 1.

In the rewarming step, as shown in FIG. 4, after the cryo-ablating is completed, the three three-way valves are adjusted to make the cold carrier medium flow in accordance with the following path: the cold carrier medium first flows out from the storage tank 1, through the flowmeter, the pumping device 20, the flow rate regulation valve and then into the heating device 16, and after being heated to 37°C by the heating device 16, the cold carrier medium flows through the rewarming pipe 15 into the catheter 7, then heats and rewarms the tissue after ablation, then flows out of the catheter 7, through the third three-way valve 19, through the rewarming backflow pipe 18 and finally back into the storage tank 1.

Apparently, the afore-mentioned embodiments are merely examples illustrated for clearly describing the present invention, rather than limiting the implementation ways thereof. For a person skilled in the art, various changes and modifications in other different forms can be made on the basis of the afore-mentioned description. It is unnecessary and impossible to exhaustively list all the implementation ways herein. However, any obvious changes or modifications derived from the afore-mentioned description are intended to be embraced within the protection scope of the present invention.

## Claims

1. A cryoablation apparatus, **characterized in** comprising:
a cryoballoon (8), provided with a circulating cold carrier medium therein, adapted for contacting a human tissue and performing cryoablation on the human tissue;
a catheter (7), connected to the cryoballoon (8), having an inlet end and an outlet end, and adapted for transporting the cold carrier medium into and out of the cryoballoon (8);
a storage tank (1), provided with the cold carrier medium stored therein;
a delivery pipeline (2), having one end communicated with the storage tank (1) and another end communicated with the inlet end of the catheter (7);
a recovery pipeline (3), having one end communicated with the storage tank (1) and another end communicated with the outlet end of the catheter (7); and
a refrigeration assembly, connected in series with the delivery pipeline (2), and adapted for cooling the cold carrier medium in the delivery pipeline (2).

2. The cryoablation apparatus according to Claim 1, **characterized in that**, the refrigeration assembly comprises:
a first cold energy exchanger (5), mounted on the delivery pipeline (2) and used for cooling the cold carrier medium flowing through the first cold energy exchanger (5) by heat exchange;
a cold energy generator (4), for generating cold energy and providing the cold energy to the first cold energy exchanger (5).

3. The cryoablation apparatus according to Claim 2, **characterized in that**, the refrigeration assembly further comprises:
a second cold energy exchanger (6), having a hot fluid channel mounted on the delivery pipeline (2) and a cold fluid channel mounted on the recovery pipeline (3), wherein, cold energy exchange occurs between the cold fluid channel and the hot fluid channel to precool the cold carrier medium flowing through the hot fluid channel, and the hot fluid channel is connected between the storage tank (1) and the first cold energy exchanger (5).

4. The cryoablation apparatus according to Claim 2, **characterized in** further comprising:
a bypass pipe (9), communicated with the delivery pipeline (2) and the recovery pipeline (3) to allow the delivery pipeline (2) and the recovery pipeline (3) to form a precooling looping path connecting the storage tank (1) and the first cold energy exchanger (5) in series;
wherein, the bypass pipe (9) is communicated with the delivery pipeline (2) via a first three-way valve (10).

5. The cryoablation apparatus according to Claim 3, **characterized in that**, the refrigeration assembly further comprises:
a cold storage device (11), mounted on the recovery pipeline (3), communicated with the first cold energy exchanger (5) via the bypass pipe (9), and adapted for storing cold energy coming from the first cold energy exchanger (5).

6. The cryoablation apparatus according to Claim 5, **characterized in that**, the refrigeration assembly further comprises:
a heat-insulation device (12), having a heat-insulation chamber (13) adapted for reducing or eliminating heat conduction to the exterior thereof, with the first cold energy exchanger (5), the second cold energy exchanger (6), the cold storage device (11) and a cold energy output end of the cold energy generator (4) located inside the heat-insulation chamber (13).

7. The cryoablation apparatus according to Claim 6, **characterized in that**, the heat-insulation device (12) is a box mounted with a vacuumizing device (14) communicated with the heat-insulation chamber (13).

8. The cryoablation apparatus according to Claim 6, **characterized in that**, the heat-insulation device (12) is a box with the heat-insulation chamber (13) thereof filled with heat insulation substance.

9. The cryoablation apparatus according to Claim 2, **characterized in** further comprising a rewarming looping path for transporting the cold carrier medium in the storage tank (1) to the inlet end of the catheter (7) of the cryoablation apparatus.

10. The cryoablation apparatus according to Claim 9, **characterized in that**, the rewarming looping path comprises:
a rewarming pipe (15);
wherein, an inlet end of the rewarming pipe (15) is connected by a second three-way valve (17) to a side of the delivery pipeline (2) that is located upstream of the first cold energy exchanger (5).

11. The cryoablation apparatus according to Claim 2, **characterized in** further comprising a rewarming looping path for transporting the cold carrier medium in the storage tank (1) to the inlet end of the catheter (7) of the cryoablation apparatus after heating the cold carrier medium.

12. The cryoablation apparatus according to Claim 11, **characterized in that**, the rewarming looping path comprises:
a rewarming pipe (15) with a heating device (16) connected in series;
wherein, an inlet end of the rewarming pipe (15) is connected by a second three-way valve (17) to an upstream side of an inlet to the hot fluid channel.

13. The cryoablation apparatus according to Claim 9, **characterized in that**, the rewarming looping path also comprises:
a rewarming backflow pipeline for connecting the outlet end of the catheter (7) of the cryoablation apparatus to the storage tank (1).

14. The cryoablation apparatus according to Claim 13, **characterized in that**, the rewarming backflow pipeline comprises:
a rewarming backflow pipe (18) having both ends thereof communicated with the recovery pipeline (3) and being connected in parallel to the second cold energy exchanger (6);
wherein, an inlet of the rewarming backflow pipe (18) is connected by a third three-way valve (19) to the delivery pipeline (2).

15. The cryoablation apparatus according to Claim 14, **characterized in that**, the refrigeration assembly further comprises:
a heat-insulation device (12), having a heat-insulation chamber (13) adapted for reducing or eliminating heat conduction to the exterior thereof, with the first cold energy exchanger (5), the second cold energy exchanger (6), a cold storage device (11) and a cold energy output end of the cold energy generator (4) located inside the heat-insulation chamber (13);
wherein, the rewarming backflow pipe (18) is located outside the heat-insulation device (12).

16. The cryoablation apparatus according to any one of Claims 1-15, **characterized in that**, a pumping device (20) is connected in series on the delivery pipeline (2) or the recovery pipeline (3), the pumping device (20) is adapted for providing motive power for the flowing of the cold carrier medium.

17. A cryoablation method, **characterized in** comprising:
a precooling step, in which a cold carrier medium is circulated through a cold energy generator (4) for cooling;
an ablating step, in which the precooled cold carrier medium is re-circulated through the cold energy generator (4) for re-cooling and then is transported to a target tissue of a human body, so that cold energy exchange occurs between the cold carrier medium and the target tissue to cool the target tissue for cryoablation of the target tissue;
a recovering step, in which the cold carrier medium after cold energy exchange with the target tissue is transported out of the human body and into a storage tank; and
a rewarming step, in which the transportation of the cooled cold carrier medium into the human body is stopped, and the target tissue is rewarmed.

18. The cryoablation method according to Claim 17, **characterized in that**, in the recovering step, the cold carrier medium after cold energy exchange with the target tissue is transported out of the human body, and residual cold energy in the cold carrier medium transported out of the human body is utilized to carry out cold energy exchange with the cold carrier medium flowing out of the storage tank at a second cold energy exchanger (6), so as to cause the cold carrier medium before entry into the first cold energy exchanger (5) to be cooled and then transported into the first cold energy exchanger (5).

19. The cryoablation method according to Claim 17, **characterized in that**, a part of cold energy is stored in the precooling stage, and then transferred to the cold carrier medium recovered from a catheter (7), so as to be exchanged to the cold carrier medium flowing out of the storage tank at a second cold energy exchanger (6).

20. The cryoablation method according to Claim 17, **characterized in that**, in the rewarming step, the cold carrier medium is circulated through a heating device (16) for being warmed, and then the cold carrier medium after being warmed is transported to the target tissue of the human body, so as to warm the cooled target tissue by heat exchange between the cold carrier medium and the target tissue.

21. The cryoablation method according to Claim 17, **characterized in that**, in the rewarming step, uncooled cold carrier medium is transported to the human body, so as to warm the target tissue by heat exchange between the uncooled cold carrier medium and the target tissue.
